# EUROPÄISCHE PATENTSCHRIFT

(11) **EP 1 722 682 B1**
(45) Veröffentlichungstag und Bekanntmachung des Hinweises auf die Patenterteilung: **29.06.2011**
(21) Anmeldenummer: 05714935.3
(22) Anmeldetag: 04.02.2005
(51) Int. Cl.: A61B 5/085

(54) **GERÄT ZUR OSZILLOMETRISCHEN ANALYSE DER ATEMWEGIMPEDANZ**
APPLIANCE FOR THE OSCILLOMETRIC ANALYSIS OF THE IMPEDANCE OF THE RESPIRATORY TRACT
APPAREIL POUR ANALYSE OSCILLOMETRIQUE DE L'IMPEDANCE DES VOIES RESPIRATOIRES

(30) Priorität: 14.02.2004 DE 102004008057
(43) Veröffentlichungstag der Anmeldung: 22.11.2006
(73) Patentinhaber: Ganshorn, Peter, 97618 Niederlauer (DE)
(72) Erfinder: Ganshorn, Peter, 97618 Niederlauer (DE)
(74) Vertreter: Pöhner, Wilfried Anton
(86) Internationale Anmeldenummer: PCT/DE2005/000184
(87) Internationale Veröffentlichungsnummer: WO 2005/077270

(56) Entgegenhaltungen:
- EP-A- 0 373 585
- DD-A1- 225 910
- DE-A1- 1 961 539
- DE-A1- 2 117 847
- DE-A1- 4 326 374
- US-A1- 2002 159 606
- LOPES DE MELO P ET AL: "LINEAR SERVO-CONTROLLED PRESSURE GENERATOR FOR FORCED OSCILLATION MEASUREMENTS" MEDICAL AND BIOLOGICAL ENGINEERING AND COMPUTING, PETER PEREGRINUS, STEVENAGE, GB, Bd. 36, Nr. 1, 1998, Seiten 11-16, XP000727963 ISSN: 0140-0118

## Beschreibung

Die Erfindung betrifft ein Gerät zur Bestimmung der Atemwegimpe- , danz (Z_{aw}) durch Messung des Wechseldrucks (dp) am Mund eines Patienten nach Erzeugung eines oszillierenden Luftdrucksignals, bestehend aus einem Mundstück, einem elektroakustischen Wandler mit einer beweglichen, steifen Membran als mechanischem Schwingungssystem zur Erzeugung des oszillierenden Luftdrucksignals, einem Verbindungsschlauch vom elektroakustischen Wandler zum Mundstück, einem Referenzwiderstand zur Bestimmung der Referenzimpedanz (Z_{ref}), und einer Recheneinrichtung zur Berechnung der Atemwegimpedanz (Z_{aw}) aus der Referenzimpedanz (Z_{ref}) des Referenzwiderstandes und der Gesamtimpedanz (Z_{ges}) sowie dem Gesamtphasenwinkel (φ).

Die Leistungsfähigkeit einer Lunge wird bestimmt durch die Effektivität beim Gasaustausch, d.h. bei der Abgabe von Sauerstoff an das Blut bzw. der Aufnahme von Kohlendioxid aus dem Blut. Beide Vorgänge sind von einer Vielzahl von Parametern abhängig, wie z.B. dem Lungenvolumen, der Verteilung der Atemluft innerhalb der Lunge, der Oberflächengröße der alveolokapillaren Membranen, deren Stärke und Diffusionseigenschaften, oder der Ventilation. In all diesen Parametern können, bei einer Abweichung der Parameter von der Norm, auch Ursachen für Störungen der Lungentätigkeit begründet sein.

Von besonderem medizinischem Interesse ist jedoch der Widerstand der Atemwege. Ein erhöhter Atemwegs-Widerstand ist einer der wesentlichen Indices für eine abnormale Funktion der Lunge, wie Asthmatiker und Allergiker beispielshaft belegen. Mit der Bestimmung der "Atemwegsimpedanz" hat der Arzt einen, wenn nicht "den" Basisparameter der Lungenfunktion. Alle bekannten Verfahren zur Bestimmung des Atemwegswiderstandes, wie Bodyplethysmographie, Fluß/Volumen Bestimmung, Verschlussdruck Resistance u.a.m. sind indirekte Meßmethoden, die eine nicht unerhebliche Kooperation des Patienten erfordern. Alleine die Bestimmung des A-temwegswiderstanden mit Hilfe der oszillatorischen Methode erlaubt eine direkte Messung des Atemwegswiderstandes ohne Mitarbeit des Patienten.

Der Begriff der Impedanz ist aus der Elektrizitätslehre bekannt und bezeichnet den Stromwiderstand in einem Wechselstromkreis (Wechselstromwiderstand). In einer elektrischen Schaltung mit zwei Polen, an denen ein Wechselstrom anliegt, ist der Wechselstrom gegenüber der anliegenden Spannung um einen bestimmten Phasenwinkel verschoben. Die physikalische exakte Beschreibung der Phasenverschiebung ist mit Hilfe komplexer Zahlen möglich. Für eine komplexe Zahl z gilt in der algebraischen Darstellung z = x + iy , in der exponentiellen Darstellung gilt z = r e^{iϕ} und in der trigonometrischen Darstellung gilt z = r (cos ϕ + i sin ϕ). Eine komplexe Zahl wird durch den Betrag r = |Z| und den Phasenwinkel ϕ bestimmt. Im Fall der respiratorische Impedanz Zᵣₑₛ, die als Widerstand eine komplexe Größe darstellt und auch als Gesamtimpedanz (Z_{aw}) bezeichnet wird, haben Modellversuche gezeigt, dass Zᵣₑₛ durch diese beiden Größen auch physikalisch ausreichend definiert ist.

Die Widerstände des Atemtrakts können in Analogie zu den Widerständen eines elektrischen Schaltkreises bezeichnet werden. Der reelle Widerstand der Atmung ist immer positiv und wird durch die Reibungsverluste in Gas und Gewebe verursacht. Der reelle Atemwiderstand wird auch als Resistance (Strömungswiderstand) bezeichnet und entspricht dem Realteil (x) des komplexen Atemwiderstandes. Trachea und die Bronchien wirken wegen der Massenträgheit des darin enthaltenen Gases als induktive Widerstände. Aufgrund der Kompressibilität der Luft, sowie aufgrund der Gewebeelastizität tritt auch eine kapazitive Widerstandskomponente auf. Die Summe (im Sinne eine vektoriellen Addition) aus den induktiven und den kapazitiven Widerständen ist der Blindwiderstand, der auch als Reaktance bezeichnet wird und den ImaginärRealteil (y) des komplexen Widerstandes ausmacht. Die Resistance sowie die Reactance wird in [kPa/(I/s)] angegeben. Die Bezeichnung Resistance oszillation (Ros) entspricht der respiratorische Impedanz Zᵣₑₛ bei einer Oszillationsfrequenz.

Da die zur Analyse komplexer Zahlen erforderlichen Rechenvorrichtungen erst später.zur Serienreife gelangten bezog sich die Auswertung der im Rahmen der oszillatorischen Widerstandsmessung ermittelten Daten zunächst nur auf die Bestimmung des Atemwiderstandes als reale Größe (DE 1960 640), die auch als oszillatorische Resistance (Ros) bezeichnet wird.

Aus dem Stand der Technik sind verschiedene Methoden zur oszillatorischen Bestimmung des Atemwegwiderstandes bekannt. Dabei werden dem Atemtrakt des Patienten über den Mund bei offener oder geschlossener Nase Oszillationen von 4 bis zu 50 Hz aufgezwungen.

Das Gerät "SIREGNOST FD5" der Firma Siemens lagert der menschlichen Atemfrequenz von 0,2 bis 0,3 Hz einen höher frequenten Oszillationsstrom von ca. 10 Hz auf. Der aufgelagerte Oszillationsstrom von 10 Hz wird durch eine ventillose Membranpumpe mit einem definierten Hubvolumen erzeugt. Ein zusätzlich am Mundstück angeschlossener Plastikschlauch mit bekanntem Radius und bekannter Länge wird dabei parallel zum Atemtrakt des Patienten oszilliert. Der Schlauch dient als Referenzwiderstand im Sinne einer Impedanz, die durch die Induktivität der oszillierenden Luftsäule verursacht wird. Über diesen Referenz-Schlauch kann der Patient ungehindert atmen, wobei das Volumen des Schlauchs den Totraum nur unwesentlich vergrößert. Der oszillierende Volumenstrom erzeugt im Mund einen Wechseldruck (dp), der durch ein Mikrophon aufgenommen wird. Der Wechseldruck weist gegenüber dem vom Generator des Luftdrucksignals ausgehenden Volumenstrom eine Phasenverschiebung auf (φ). Nach Filterung, Gleichrichtung und Glättung des analogen Spannungssignals wird die Resistance am Instrument angezeigt.

Die Berechnung des am Zeigerinstrument ablesbaren Wertes erfolgt durch eine Recheneinrichtung, die geeignet ist, die Atemwegimpedanz (Z_{aw}) aus der Referenzimpedanz (Z_{ref}) und der Gesamtimpedanz (Z_{ges}) sowie dem Gesamtphasenwinkel (φ) zu errechnen. Für den Zusammenhang zwischen der Gesamtimpedanz (Z_{ges}) und Atemwegimpedanz (Z_{aw}) sowie der Referenzimpedanz (Z_{ref}) gilt 1/Z_{ges}= 1/ Z_{aw} + 1/ Z_{ref}., wenn Z_{aw} und parallel Z_{ref} geschaltet sind. Durch Anwendung der Rechenvorschriften für komplexe Zahlen kann der Realteil und der Imaginärteil der Atemwegimpedanz (Z_{aw}) bestimmt werden. Die Referenzimpedanz (Z_{ref}) ist durch den Referenz-Luftschlauch vorgegeben, während die Gesamtimpedanz (Z_{ges}) durch einen Druckaufnehmer gemessen wird, wobei zu bedenken ist, dass der Volumenstrom dV/dt konstant bleibt. Aufgrund dieser Konstanz kann der gemessene Wechseldruck am Mund als Maß für die Gesamtimpedanz (Z_{ges}) gesehen werden. Die Recheneinrichtung berechnet die Atemwegimpedanz (Z_{aw}) sowie den Realteil der Atemwegimpedanz (Z_{aw}) und den Atemtraktphasenwinkel aus der vorgegebenen Referenzimpedanz (Z_{ref}) und dem vorgegebenen Referenzphasenwinkel sowie dem gemessenen Wechseldruck am Mund als Maß für die Gesamtimpedanz (Z_{ges}) und den gemessenen Gesamtphasenwinkel.

Eine derartige Vorrichtung zur Bestimmung des Atemwegswiderstandes ist in Dokument EP 0373 585 beschrieben.

Aus dem Stand der Technik ist außerdem die Impuls-Oszillometrie (IOS) bekannt, bei der ein elektrischer Rechteck-Impuls durch die mechanischen Eigenschaften des nachgeschalteten Lautsprechers in ein Gemisch der gewünschten Frequenzen umgewandelt wird. Ein nach dem Prinzip der Impuls-Oszillometrie arbeitendes Lungenfunktionsprüfungsgerät besteht aus einem Lautsprecher zum Erzeugen der Testimpulse, einem T-förmigen Verbindungsstück, in dem die Modulation des Atemstroms durch das Testsignal erfolgt. Eine vom Lautsprecher wegführende Öffnungen der T-Verbindung weist einen zur Umgebungsluft definierten Widerstand auf, über den der Patient frei atmen kann. Die andere vom Lautsprecher wegführende Öffnung des T-Stücks ist mit einem Pneumatograph, in den ein Drucksensor integriert ist, verbunden. Während der Drucksensor die Messung des Munddrucks (p) erlaubt wird durch den Pneumatographen der Volumenstrom (V') gemessen. Die Messergebnisse werden als elektrische Signale ausgelesen. Der Druck-Flow-Quotient ist die zu bestimmende thorako-pulmonale Impedanz des Patienten. Zum Kalibrieren des Messkopfs wird am Ausgang des Drucksensors, der dem Pneumographen nachgeschaltet ist, die Referenzimpedanz (Z_{ref}) gemessen. Zu diesem Zweck wird ein Siebwiederstandselement, dessen Form verschieden gestaltet sein kann, gemessen. In Dokument DE 432 63 74 A1 ist eine konisch aufgeweitete Referenzimpedanz mit Siebwiderstand beschrieben, die sich für die Kalibrierung der Druck- und Flow- Messeinrichtung eines Gerätes zur oszillometrischen Messung des Atemwegwiederstandes eignet.

Die Aufgabe der Erfindung besteht in der Bestimmung der Atemwegimpedanz Z_{aw} bzw. Zᵣₑₛ bei einem Patienten durch genaue Messung der Gesamtimpedanz und des dazugehörigen Phasenwinkels mit Hilfe eines Gerätes das mit nur einem mechanischen Schwingungssystem ausgestattet sein soll ist. Dabei soll die Detektion der durch den Patienten veränderten Auslenkung des mechanischen Schwingungssystems indirekt und berührungslos erfolgen, so dass das Schwingungssystem durch die Messung nicht gestört wird.

Die Aufgabe wird gelöst durch die Konstruktion eines Gerätes zur Messung der respiratorischen Impedanz, das dadurch gekennzeichnet ist, dass die durch den Wechseldruck (dp) der Patientenatmung verursachte Änderung der Auslenkung des mechanischen Schwingungssystems am elektroakustischen Wandler als Druckänderung im geschlossenen Raum hinter der Membrane durch eine Messvorrichtung berührungslos messbar ist.

Das erfindungsgemäße Gerät dient der Errechnung des komplexen Atemwegwiderstands Z_{aw} bzw. Zᵣₑₛ und besteht im wesentlichen aus einem elektroakustischen Wandler dessen mechanisches Schwingungssystem zur Erzeugung eines oszillierenden Luftdrucksignals dient. Das Luftdrucksignal wird über einen Verbindungsschlauch zur Atemwegsmaske, die der Patient trägt, geführt. Mit dem Verbindungsschlauch ist auch ein Referenzwiderstand verbunden, der zur Bestimmung der Referenzimpedanz (Z_{ref}) dient. Die mit dem Referenzwiderstand und dem elektroakustischen Wandler sowie mit dem mechanischen Schwingungssystem verbundene Recheneinrichtung eignet sich zur Durchführung von Rechenoperationen mit komplexen Zahlen als Eingangsgrößen wie z.B. der Fouriertransformation (FFT), mit deren Hilfe die in der Zeitdomaine aufgenommen Daten in die Frequenzdomäne umgerechnet werden können. Die Details der Fouriertransformation sind dem Fachmann bekannt.

Der Kerngedanke der Erfindung besteht darin, dass der durch die Patientenatmung verursachte Wechseldruck (dp) auf das mechanische Schwingungssystem des elektroakustischen Schallwandlers wirkt, der als Lautsprecher dient. Das als Impuls (Lautsprecher) dienende mechanische Schwingungssystem, das den Volumenstrom dV/dt erzeugt, ist auch den durch die Patientenatmung verursachten Druckschwankungen (dp) ausgesetzt. Zur Erfassung der Druckschwankungen werden die Auslenkungen des mechanischen Schwingungssystems gemessen. Aufbauend auf dieser Messung wird der für die Resistance-Berechnung benötigte effektive Volumenstrom dV/dt als Funktion der Membranbewegung direkt bestimmbar.

Zum Nachweis des Einflusses der Patientenatmung auf das Schwingungssystem des elektroakustischen Wandlers sind je nach dessen Prinzip induktive Messmethoden bekannt und kapazitive, piezoelektrische oder optische Messungen für den Fachmann naheliegend.

Das wesentliche Merkmal der Erfindung ist der Nachweis des Einflusses der Patientenatmung durch die Messung der entstehenden Druckveränderungen in dem geschlossenen Raum hinter der Membran. Zum Vergleich werden im Folgenden auch noch die bekannte und die naheliegenden Messmethoden geschildert.

Es gehört zum Wesen der Erfindung, dass die durch die Patientenatmung verursachten Änderungen der Auslenkung des mechanischen Schwingungssystems zeitgleich mit der Erzeugung des oszillierenden Luftdrucksignals erfolgt. Aufgrund der zeitgleichen Detektion kommt es am mechanischen Schwingungssystem zu der erwähnten Überlagerung der Schwingungen des erzeugten Luftdrucksignals und jener durch den Wechseldruck der Patientenatmung verursachten Schwingungen.

Das erfindungsgemäße Geräte weist als mechanisches Schwingungssystem eine flexible Membran, die aus einem feuchtigkeitsresistenten Material oder aus einem metallischen Blech besteht. Das Material der Membran soll so beschaffen sein, dass es die Erzeugung und Erfassung niederfrequenter Schwingungen, so wie sie für den Einsatz des erfindungsgemäßen Gerätes erforderlich sind, erlaubt.

Der sowohl zur Erzeugung als auch zur Detektion von Schall eingesetzte elektroakustische Wandler ist das Herzstück des erfindungsgemäßen Geräts und beinhaltet verschiedene Ausführungsformen. Schall besteht aus Schwingungen, die in der Luft Dichteschwankungen hervorrufen und dadurch übertragen werden. Lautsprecher sind elektroakustische Wandler, die den Schallwechseldruck über ein mechanisches Schwingungssystem (Membran) in eine elektrische Spannung umwandeln können.

Die Membran als aktives Element wird beim Lautsprecher durch elektrische Wechselströme in mechanische Schwingung versetzt und erzeugt dadurch Schallwellen. Umgekehrt kann das mechanische Schwingungssystem zur Umwandlung von Schall in tonfrequente Spannungen und Ströme dienen. Die verschiedenen elektrostatischen Wandler unterscheiden sich im Absolutwert und in der Frequenzabhängigkeit des Wandlerwirkungsgrades, aber auch in ihrer mechanischen Empfindlichkeit und in ihrer Schalldruckbelastbarkeit.

Als elektroakustische Wandler sind auf aktuellem Stand der Technik elektrodynamische, elektromagnetische und piezoelektrische Wandler bekannt.

Beim elektrodynamischen Wandler bewegt die Membran eine Spule in einem Topfmagneten, so dass mit der Bewegung in der Schwingspule tonfrequente elektrische Wechselspannungen induziert werden, die zur Schallwelle proportional sind. Fließt durch die Schwingspule eines Lautsprechers ein tonfrequenter Wechselstrom, so bewegt sich die Spule in Folge der Induktion axial im Luftspalt und zwar im Rhythmus des Wechselstroms. Die an der Schwingspule befestigte Membran wird mit bewegt und regt dabei die umgebende Luft zu Schallschwingungen an. Eine Variante des dynamischen Mikrofons mit Schwingspule ist das Bändchenmikrofon, bei dem Membran und Tauchspule durch ein geriffeltes Aluminiumbändchen ersetzt sind, das im Rhythmus der Schallwellen im Magnetfeld schwingt.

Beim elektromagnetischen Wandler verändert die Bewegung der magnetischen Membran den Luftspalt eines Magneten, so dass der magnetische Fluss im Magnetjoch moduliert wird und in einer Wicklung eine elektrische Spannung induziert wird.

Beim piezoelektrischen Wandler bewirkt die Deformation eines Kristalls mit piezoelektrischen Eigenschaften eine Verschiebung der Ladungsstruktur und der piezoelektrisch erzeugten Oberflächenladung, deren elektrische Spannung zum Schalldruck proportional ist. Umgekehrt zeigen piezoelektrische Kristalle bei elektrischer Aufladung eine Dickenänderung, die in tonfrequente Schwingungen der Membran umsetzbar ist.

Die geschilderten Merkmale der in ihrem Prinzip bekannten verschiedenen Wandler-Typen legen dem Fachmann nahe, dass die Auslenkung der Membran in Abhängigkeit vom eingesetzten Wandler induktiv, kapazitiv, piezoelektrisch oder optisch gemessen werden kann.

Als eine Ausführungsform einer optischen Messung wird die durch die Patientenatmung verursachte Änderung der Auslenkung des mechanischen Schwingungssystems durch einen Laser detektiert. Zu diesem Zweck sind auf der Membran an einer oder mehreren Stellen Reflektoren oder Detektoren angebracht. Der Strahl des ortsfesten Lasers erfasst die Bewegung der Membran und detektiert dadurch deren Schwingungen. Die technischen Details dieser Messung sind dem Fachmann bekannt.

Im Unterschied zu diesen bekannten oder für den Fachmann naheliegenden Messmethoden ist es das wesentliche, kennzeichnende Merkmal der Erfindung, dass die Auslenkung des mechanischen Schwingungssystems am elektroakustischen Wandler als Druckänderung im geschlossenen Raum hinter der Membrane durch eine Messvorrichtung berührungslos gemessen wird.

Der wesentliche Vorteil ist, dass das Schwingungssystem durch diese Messmethode überhaupt nicht gestört wird.

Beim erfindungsgemäßen Gerät sind für die Gestaltung des Referenzwiderstandes im wesentlichen zwei alternative Ausführungsformen denkbar. Die erste Ausführungsform besteht aus einem zylindrischen Luftschlauch dessen Innenwiderstand mit zunehmender Länge größer wird. Eine alternative Ausführungsform betrifft einen Luftschlauch der an dem vom Mundstück abgewandten Ende konisch aufgeweitet ist, wobei in diese Öffnung ein Siebwiderstand eingelassen ist. Die Vorteile und Besonderheiten dieser Ausführungsform ergeben sich aus DE 432 63 74 A1. Es ist vorgesehen, dass die Referenzwiderstände zum Zweck der Hygiene abnehmbar und austauschbar sind.

In einer besonderen Ausführungsform ist die Atemwegsmaske so gestaltet, dass der Außendurchmesser in dem Bereich, in dem die Atemwegsmaske in den Verbindungsschlauch eingeführt wird, geringer ist, als der Innendurchmesser des Verbindungsschlauches. Die Atemwegsmaske liegt auf diese Weise mit leichter Spannung auf der Innenseite des Verbindungsschlauches an, so dass die beiden Bestandteile des erfindungsgemäßen Gerätes luftdicht miteinander verbunden sind.

In einer alternativen Weiterbildung der Atemwegsmaske weist der in den Verbindungsschlauch einführbare Teil seitlich zwei oder mehrere Öffnungen auf. Die Öffnungen sind zueinander versetzt, so dass sie sich nicht direkt gegenüber liegen. Die Öffnungen können durch ein perforiertes flüssigkeitsaufsaugendes Material abgedeckt sein. Das Material ist vorzugsweise auf der Innenseite der Atemwegsmaske angebracht.

In einer Weiterbildung des erfindungsgemäßen Gerätes ist vorgesehen, dass der Recheneinheit ein Bildschirm oder ein Ausgabegerät in Form eines Druckers zugeordnet ist. Die Messwerte (Resistance, Reactance oder Phasenwinkel) können in diesem Fall in einem Diagramm aufgetragen und am Bildschirm graphisch aufbereitet und analysiert werden. Ein an das erfindungsgemäße Geräte angeschlossener Drucker erlaubt das Ausdrucken der Daten.

Im folgenden sollen weitere Einzelheiten und Merkmale der Erfindung anhand eines Beispiels näher erläutert werden. Das abgebildete Beispiel soll die Erfindung jedoch nicht einschränken, sondern nur erläutern.

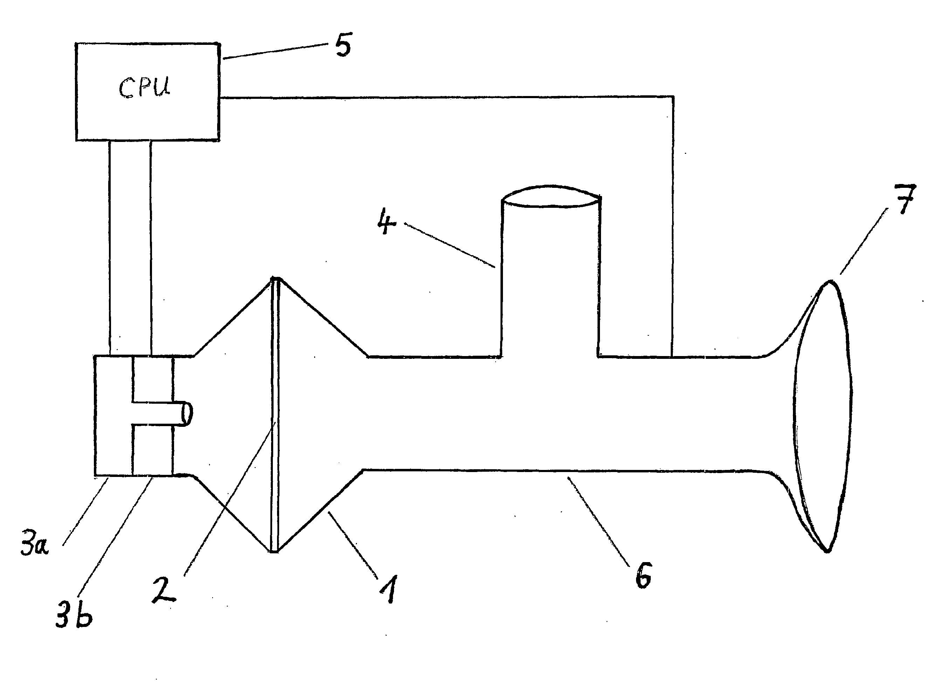

Figur 1 zeigt in schematischer Darstellung den Aufbau des erfindungsgemäßen Gerätes zur Bestimmung der Atemwegsimpetanz. Der in technischer Hinsicht wesentlichste Bestandteil ist der elektroakustische Wandler 1 mit dem mechanischen Schwingungssystem 2, das sowohl zur Erzeugung eines oszillierenden Luftdruckssignals, als auch zur Detektion des durch die Patientenatmung verursachten Wechseldrucks (dp) dient. Der durch die Patientenatmung verursachte Wechseldruck (dp) geht vom Patienten aus, dessen Mund am Mundstück 7 anliegt. Die Verbindung zwischen dem abnehmbaren Mundstück 7 und dem elektroakustischen Wandler 1 wird durch den Verbindungsschlauch 6 hergestellt. Eine Abzweigung vom Verbindungsschlauch 6 bildet der abnehmbare und austauschbare Referenzwiderstand 4. In der einfachsten Ausführungsform kann der Referenzwiderstand 4 aus einem zylindrischen Schlauch bestehen, durch den der Patient frei atmen kann. Die Impedanz des schlauchförmigen Referenzwiderstands 4 wird durch die Induktivität der oszillierenden Luftsäule verursacht. Über den Referenzwiderstand 4 kann der Patient ungehindert atmen, ohne dass das Volumen des Schlauches den Totraum wesentlich vergrößert. Der durch die Atmung des Patienten verursachte Wechseldurck (dp) versetzt das mechanische Schwingungssystem 2, das sogleich zur Erzeugung des oszillierenden Luftdrucksignal dient, in Schwingungen. Im Vergleich zu dem zunächst erzeugten oszillierenden Luftdrucksignal kommt es durch die Überlagerung der Schwingungen zu einer Abweichung, welche die Atmung des Patienten charakterisiert und durch die weiteren Bestandteile des Mikrophons 3b detektiert wird.

Verbunden mit dem Generator des oszillierenden Luftdrucksignals, der als Lautsprecher ausgeführt ist, und den Mikrophonbestandteilen 3b sowie mit dem Referenzwiderstand 4 ist eine zentrale Recheneinheit. Die zentrale Recheneinheit erlaubt die Steuerung des Lautsprechers 3a und die Erfassung der durch das Mikrophon 3b erfassten Daten sowie deren Auswertung. Die Recheneinheit arbeitet unter Berücksichtigung der komplexen Rechenregeln, die dem Fachmann bekannt sind. Nicht dargestellt ist der an die zentrale Recheneinheit angeschlossene Bildschirm und/oder Drucker.

## Patentansprüche

1. Gerät zur Bestimmung der Atemwegimpedanz Z_{aw} durch Messung des Wechseldrucks (dp) am Mund eines Patienten nach Erzeugung eines oszillierenden Luftdrucksignals, bestehend aus
- einem Mundstück (7),
- einem elektroakustischen Wandler (1) mit einer beweglichen,
steifen Membran als mechanischem Schwingungssystem (2) zur Erzeugung des oszillierenden Luftdrucksignals,
- einem Verbindungsschlauch (6) vom elektroakustischen Wandler (1) zum Mundstück (7),
- einem Referenzwiderstand (4) zur Bestimmung der Referenzimpedanz Z_{ref}, und
- einer Recheneinrichtung (5) zur Berechnung der Atemwegimpedanz Z_{aw} aus der Referenzimpedanz Z_{ref} des Referenzwiderstandes (4) und der Gesamtimpedanz Z_{ges} sowie dem Gesamtphasenwinkel φ,
**dadurch gekennzeichnet, dass** die durch den Wechseldruck (dp) der Patientenatmung verursachte Änderung der Auslenkung des mechanischen Schwingungssystems (2) am elektroakustischen Wandler (1) als Druckänderung im geschlossenen Raum hinter der Membrane durch eine Messvorrichtung (3b) berührungslos gemessen wird.

2. Gerät nach Anspruch 1, **dadurch gekennzeichnet, dass** die Membran aus einem feuchtigkeitsresistenten Material oder aus Blech besteht.

3. Gerät nach einem der vorhergehenden Ansprüche, **dadurch gekennzeichnet, dass** als elektroakustischer Wandler (1) ein elektrostatischer, ein elektrodynamischer, ein elektromagnetischer, ein piezoelektrischer oder ein piezoresistiver Wandler (1) eingesetzt wird.

4. Gerät nach einem der vorhergehenden Ansprüche, **dadurch gekennzeichnet, dass** der Referenzwiderstand (4) ein offen endender Luftschlauch (4) ist, der anderen Ends mit dem Mundstück (7) verbundenen ist, eine geeichte, vorbestimmbare Referenzimpedanz Z_{ref} aufweist und abnehmbar ist.

5. Gerät nach einem der vorhergehenden Ansprüche, **dadurch gekennzeichnet, dass** der den Referenzwiderstand (4) bildende Luftschlauch zylindrisch ist oder sich an dem vom Mundstück abgewandten Ende konisch aufweitet und mit einem Siebwiderstand verbunden ist.

6. Gerät nach einem der vorhergehenden Ansprüche, **dadurch gekennzeichnet, dass** als Mundstück (7) eine Atemmaske verwendet wird, die Mund- und/oder Nasenöffnungen des Patienten luftdicht umschließt und durch eine luftdichte Steckverbindung mit dem Verbindungsschlauch (6) verbunden ist.

7. Gerät nach einem der vorhergehenden Ansprüche, **dadurch gekennzeichnet, dass** der Recheneinheit (5) ein Bildschirm und/oder ein Ausgabegerät in Form eines Druckers zugeordnet ist.

## Claims

1. Appliance for determining the impedance Z_{aw} of the respiratory tract by measuring the alternating pressure (dp) at the mouth of a patient after producing an oscillating air pressure signal, which comprises
- a mouthpiece (7)
- an electroacoustic transducer (1) provided with a mechanical oscillation system (2) for generating the oscillating air pressure signal,
- a connecting tube (6) from the electroacoustic transducer (1) to the mouthpiece (7),
- a reference resistance (4) for determining the reference impedance Z_{ref}, and
- a computing device (5) for calculating the impedance Z_{aw} of the respiratory tract on the basis of the reference impedance Z_{ref} of the reference resistance (4), the total impedance Zₜₒₜ, and the entire phase angle ϕ,
**characterised in that** the change in the deflection of the mechanical oscillation system (2) on the electroacoustic transducer (1), caused by the alternating pressure (dp) of the breathing of the patient, can be measured in a contactless manner by means of a measuring device (3b) as a pressure change in the closed space behind the membrane.

2. Appliance according to claim 1, **characterised in that** the membrane consists of a moisture-resistant material or of sheet metal.

3. Appliance according to one of the preceding claims, **characterised in that**, as electroacoustic transducer (1), an electrostatic, an electrodynamic, an electromagnetic, a piezoelectric or a piezoresistive transducer (1) is used.

4. Appliance according to one of the preceding claims, **characterised in that** the reference resistance (4) is an openended air tube (4), of which the other end is connected to the mouthpiece (7), has a calibrated, predeterminable reference impedance Z_{ref} and is removable.

5. Appliance according to one of the preceding claims, **characterised in that** the air tube forming the reference resistance (4) is cylindrical or is conically enlarged at that end facing away from the mouthpiece, and is connected to a sieve resistance.

6. Appliance according to one of the preceding claims, **characterised in that**, as mouthpiece (7), a respiration mask is used, which encloses the mouth and/or nose openings of the patient in an airtight manner, and is connected to the connecting tube (6) by means of an airtight plug connection.

7. Appliance according to one of the preceding claims, **characterised in that** a display screen and/or an output unit in the form of a printer is assigned to the computer unit (5).

## Revendications

1. Appareil destiné à déterminer l'impédance des voies respiratoires Z_{aw} par mesure de la pression alternative (dp) sur la bouche d'un patient après génération d'un signal de pression pneumatique oscillant, consistant en
■ un embout buccal (7),
■ un convertisseur électroacoustique (1) ayant une membrane mobile rigide (2) destinée à générer un signal de pression pneumatique oscillant,
■ un flexible de liaison (6) provenant d'un convertisseur électroacoustique (1) et allant vers l'embout buccal (7),
■ une résistance de référence (4) destinée à calculer l'impédance des voies respiratoires Z_{aw} et
■ un dispositif de calcul (5) destiné à calculer l'impédance des voies respiratoires Z_{aw} à partir de l'impédance de référence Zᵣₑₜ de la résistance de référence (4), de l'impédance totale Z_{ges} et de l'angle de phase totale ϕ,
**caractérisé par le fait que** la modification, provoquée par la pression alternative (dp) de la respiration du patient, de la direction du système de vibrations mécanique (2) sur le convertisseur électroacoustique (1) est mesurée, en tant que modification de la pression dans l'espace fermé situé derrière la membrane, par un dispositif de mesure sans contact (3b).

2. Appareil selon la revendication 1 **caractérisé par le fait que** la membrane consiste en un matériau résistant à l'humidité ou en une tôle.

3. Appareil selon une des revendications précédentes, **caractérisé par le fait qu'**un convertisseur électrostatique, électrodynamique, électromagnétique, piezoélectrique ou piezorésistif (1) est utilisé en tant que convertisseur électroacoustique (1).

4. Appareil selon une des revendications précédentes, **caractérisé par le fait que** la résistance de référence (4) est un flexible pneumatique à extrémité ouverte (4) qui est relié à l'autre extrémité avec l'embout buccal (7), présente une impédance de référence étalonnée Z_{ref}, et est amovible.

5. Appareil selon une des revendications précédentes, **caractérisé par le fait que** le flexible pneumatique formant la résistance de référence (4) est cylindrique ou s'élargit de façon conique à l'extrémité détournée sur l'embout buccal et est relié avec une résistance en tamis.

6. Appareil selon une des revendications précédentes, **caractérisé par le fait qu'**un masque respiratoire qui comprend, d'une façon étanche à l'air, les ouvertures buccales et/ou nasales du patient, et qui est relié par une liaison enfichée étanche à l'air avec le flexible de liaison (6), est employé en tant qu'embout buccal (7).

7. Appareil selon une des revendications précédentes, **caractérisé par le fait qu'**un écran et/ou un appareil de sortie sous la forme d'une imprimante est attribué une unité de calcul (5).
